# FASCICULE DE BREVET EUROPEEN

(11) **EP 1 089 674 B1**
(45) Date de publication et mention de la délivrance du brevet: **18.08.2004**
(21) Numéro de dépôt: 99915811.6
(22) Date de dépôt: 22.04.1999
(51) Int. Cl.: A61F 2/08, A61B 17/17

(54) **DISPOSITIF D'ANCRAGE D'UN LIGAMENT SUR UNE STRUCTURE OSSEUSE POUR LIGAMENTO-PLASTIE**
BEFESTIGUNGSVORRICHTUNG FÜR EIN BAND AUF EINER KNOCHENSTRUKTUR BEI EINER SEHNENPLASTIK
DEVICE FOR ANCHORING A LIGAMENT ON A BONE STRUCTURE

(43) Date de publication de la demande: 11.04.2001
(73) Titulaire: Charles, Hugues, 59420 Mouvaux (FR)
(72) Inventeur: Charles, Hugues, 59420 Mouvaux (FR)
(74) Mandataire: Hennion, Jean-Claude
(86) Numéro de dépôt international: PCT/FR1999/000955
(87) Numéro de publication internationale: WO 2000/064378

(56) Documents cités:
- WO-A-97/20522
- WO-A-97/47244
- FR-A- 2 774 582
- GB-A- 2 288 739
- US-A- 5 501 683

## Description

L'invention se rapporte à un dispositif d'ancrage d'un ligament sur une structure osseuse pour ligamento-plastie.

Un dispositif selon le préambule de la revendication 1 est connu du document WO-A-97/20522.

Il se produit parfois une rupture de la liaison entre l'extrémité d'un ligament et l'os sur lequel il était fixé initialement.

Le ligament ne pouvant se raccrocher de manière naturelle, on fait appel à des dispositifs d'ancrage qui se fixent sur la structure osseuse et sur lesquels on accroche le ligament.

Un tel dispositif d'ancrage comprend classiquement un corps longiligne dont une extrémité présente un moyen d'accrochage sur une structure osseuse et dont l'autre extrémité présente un anneau pour y fixer au moins indirectement le ligament.

Est connu par exemple un dispositif d'ancrage FR-A-2.731.610 formant harpon comprenant un corps cylindrique de révolution à la périphérie duquel sont découpées des ailettes dont les extrémités libres s'écartent progressivement du corps précité pour s'ancrer dans la structure osseuse.

Au fur et à mesure de la traction sur la tête du dispositif d'ancrage, les extrémités libres de ces ailettes pénètrent dans la structure osseuse.

La tête de ce dispositif est pourvue d'un évidement pour la fixation au moins indirecte du ligament.

Ce dispositif est introduit en force dans un puits foré dans l'os, de sorte que les languettes, s'écartant de l'axe longitudinal, empêchent tout retrait du dispositif en pénétrant dans la paroi du puits.

La résistance à la traction de ces dispositifs d'ancrage est toutefois relativement faible.

Dans un domaine médical différent qui concerne la réduction de fractures, on connaît un dispositif (FR-A-2.721.818 ou FR-A-2.737.104) se présentant sous la forme d'un grappin, c'est à dire avec, pour moyens d'accrochage, deux branches recourbées à 180° (cent quatre vingt degrés) par rapport à l'axe longitudinal du corps et dont les extrémités libres sont acérées pour pénétrer dans l'os et notamment dans la corticale.

Aprés mise en place, ces dispositifs sont coïncés de manière à exercer sur les deux parties de l'os une traction rapprochant les deux parties.

Ces dispositifs ne pourraient en aucune façon être utilisés dans la ligamento-plastie.

Un des résultats que l'invention vise à obtenir est un dispositif pour ligamento-plastie qui remédie notamment aux inconvénients précités.

A cet effet, l'invention a pour objet un dispositif d'ancrage du type précité comprenant un corps longiligne dont une extrémité présente un moyen d'accrochage sur une structure osseuse et dont l'autre extrémité présente un anneau pour y fixer au moins indirectement le ligament, ce dispositif étant caractérisé en ce que le moyen d'accrochage comprend au moins deux pattes s'étendant selon des directions divergentes et sécantes à l'axe longitudinal du corps du dispositif et chaque patte est portée par l'extrémité d'une branche longiligne et les branches longilignes constituant le corps du dispositif s'écartent progressivement l'une de l'autre depuis l'anneau auquel elles sont associées.

L'invention sera bien comprise à l'aide de la description ci-après faite, à titre d'exemple non limitatif, en regard du dessin ci-annexé qui représente schématiquement :

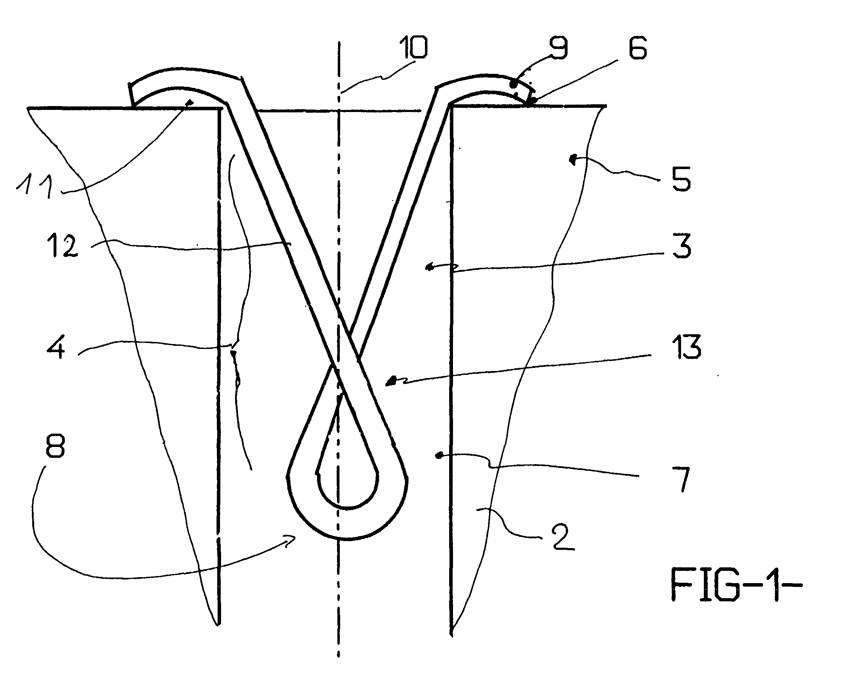

- figure 1 : un dispositif d'ancrage,

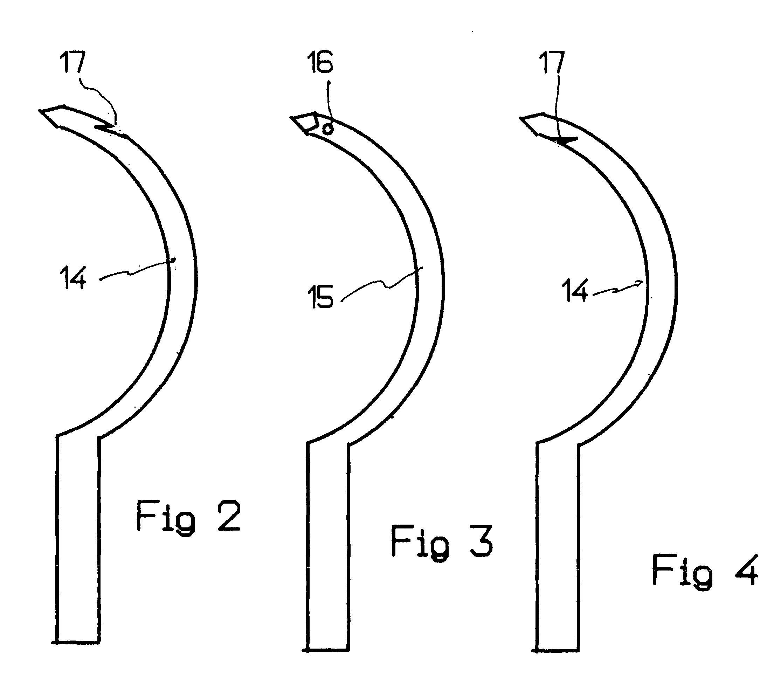

- figures 2 à 4 : différents ancillaires pour le montage.

En se reportant au dessin, on voit que pour fixer un ligament 1 sur une structure osseuse 2 telle la fixation d'un ligament sur l'épaule ou le genou, le dispositif 3 d'ancrage utilisé comprend un corps 4 longiligne dont une extrémité 5 présente un moyen 6 d'accrochage sur la structure osseuse et dont l'autre extrémité 7 présente un anneau 8 pour y fixer au moins indirectement le ligament.

Le moyen 6 d'accrochage comprend au moins deux pattes 9 s'étendant selon des directions divergentes et sécantes à l'axe 10 longitudinal du corps 4 du dispositif.

Chaque patte 9 est portée par l'extrémité d'une branche 12 longiligne et les branches 12 longilignes constituant le corps du dispositif s'écartent progressivement l'une de l'autre depuis l'anneau 8 auquel elles sont associées.

Dans une forme de réalisation, le corps du dispositif est indéformable élastiquement au moins dans le plan défini par les deux branches.

Selon un autre mode de réalisation, les branches 12 du corps comprennent un moyen 13 élastique pour permettre de rapprocher momentanément les extrémités portant les pattes et loger le tout dans un volume cylindrique de section réduite pour introduire le dispositif au travers de la peau et/ou au travers d'un alésage réalisé dans la tête de l'os.

Le corps est ainsi au moins localement élastique.

Les pattes 9 présentent chacune une face 11 d'appui tournée vers l'anneau 8 pour venir en appui sur la face externe de la structure osseuse appelée corticale.

Ces pattes sont faiblement courbées et leurs extrémités ne sont pas acérées, de sorte que l'extrémité libre des pattes ne pénètre quasiment pas dans la corticale.

En venant en simple appui sur la corticale qui est la partie la plus résistante de l'os et avec une surface d'appui relativement grande par rapport aux dispositifs existants, on obtient une plus grande résistance à la traction.

Ces faces d'appui sont présentées par des pattes 9 de section circulaire et éventuellement prismatique.

Dans le cas du corps élastique, le dispositif sera fourni aux chirurgiens dans une enveloppe en position rapprochée et se déploiera au moment de son utilisation.

Le dispositif 3 se composera de deux branches 12 se croisant pour former l'anneau auquel se fixe indirectement le ligament.

Cette particularité technique de l'anneau formé par croisement des branches confère au produit une extrême facilité pour engager le fil dans la boucle de l'anneau en glissant le fil entre les branches.

Cette conformation en anneau ouvert, par croisement des branches, répartit les contraintes mécaniques qui, par rapport à une attache en forme de U, seraient essentiellement localisées au niveau de chaque pli ou jonction entre une branche verticale et une branche horizontale.

Pour fixer la coiffe des rotateurs, le chirurgien dispose d'un ancillaire 14 comportant un trocart 15 courbe à l'aide duquel il traverse la structure osseuse.

La pointe de ce trocart courbe présente une lumière 16 permettant d'y accrocher provisoirement des fils qui vont se fixer ultérieurement, d'une part, sur l'anneau du dispositif et, d'autre part, sur les ligaments à raccrocher.

Dans une variante de réalisation, au lieu d'une lumière, on prévoit une encoche 17 à l'extérieur ou à l'intérieur de la concavité du trocart 15 courbe.

Le dispositif est introduit au travers de la peau et placé en position requise, le dispositif étant alors, par son corps logé dans le passage réalisé par l'ancillaire et les pattes disposées à l'extérieur, en appui sur la corticale.

Dans le cas d'une ligamento-plastie du genou, après avoir foré au travers de la structure osseuse un puits dont le fond comprend un canal de plus faible section, le dispositif est attiré de dehors en dedans par un fil tracteur après une incision cutanée.

Il s'applique ensuite par simple traction sur la corticale externe.

Le transplant ligamentaire est passé au préalable dans l'oeil de l'implant.

Le fil sera préalablement introduit à l'aide d'une aiguille à chas.

Les tendons demi-tendineux et droit interne forment des boucles passées au travers de l'anneau du dispositif d'ancrage.

Le puits sera alors comblé par un greffon osseux.

## Revendications

1. Dispositif (3) d'ancrage d'un ligament (1) sur une structure osseuse (2) comprenant un corps (4) longiligne dont une extrémité (5) présente un moyen (6) d'accrochage sur la structure osseuse et dont l'autre extrémité (7) présente un anneau (8) pour y fixer au moins indirectement le ligament,
- le moyen (6) d'accrochage comprenant au moins deux pattes (9) s'étendant selon des directions divergentes et sécantes à l'axe (10) longitudinal du corps (4) du dispositif et chaque patte (9) est portée par l'extrémité d'une branche (12) longiligne et les branches (12) longilignes constituant le corps du dispositif s'écartent progressivement l'une de l'autre depuis l'anneau (8) auquel elles sont associées, et
- les pattes (9) présentent chacune une face (11) d'appui tournée vers l'anneau (8) pour venir en appui sur la face externe de la structure osseuse appelée corticale, ce dispositif étant **CARACTERISE en ce que** : chaque extrémité libre des pattes (9) forme une partie de la face (11) d'appui et n'est pas acérée de sorte que l'extrémité libre de ces pattes ne pénètre quasiment pas dans la corticale.

2. Dispositif d'ancrage d'un ligament selon la revendication 1 **caractérisé en ce qu'**il est composé de deux branches (12) se croisant pour former l'anneau.

3. Dispositif d'ancrage d'un ligament selon la revendication 1 **caractérisé en ce que** le corps du dispositif est indéformable élastiquement dans le plan défini par les deux branches.

4. Dispositif d'ancrage d'un ligament selon la revendication 1 **caractérisé en ce que** les branches (12) du corps comprennent un moyen (13) élastique pour permettre de rapprocher momentanément les extrémités portant les pattes et loger le tout dans un volume cylindrique de section réduite pour introduire le dispositif au travers de la peau et/ou au travers d'un alésage réalisé dans la tête de l'os.

5. Dispositif d'ancrage d'un ligament selon la revendication 1 **caractérisé en ce que** les faces d'appui sont présentées par des pattes (9) de section circulaire.

## Patentansprüche

1. Vorrichtung (3) zur Verankerung eines Muskel/Gewebe-Bandes (1) auf einem Knochenskelett (2), wobei die Vorrichtung umfasst einen länglichen Körper (4), der an einem Ende (5) eine Einrichtung (8) zum Festhaken auf dem Knochenskelett und an dem anderen Ende (7) einen Ring bzw. eine Öse (8) aufweist, um damit mindestens indirekt das Muskel/Gewebe-Band zu fixieren, wobei
- die Einrichtung (6) zum Festhaken mindestens zwei Klauen (9) umfasst, die sich entlang der divergierenden und die Längsachse (10) des Körpers (4) der Vorrichtung schneidenden Richtungen erstrecken, wobei jede Klaue (9) an dem Ende eines länglichen Schenkels (12) angebracht ist und die länglichen Schenkel (12), die den Körper der Vorrichtung bilden, ab dem Ring bzw. der Öse (8), an dem (der) sie miteinander verbunden sind, sich allmählich voneinander entfernen, und
- die Klauen (9) jeweils eine Auflagefläche (11) aufweisen, die dem Ring bzw. der Öse (8) zugewandt ist, um auf der äußeren Oberfläche des Knochenskeletts, als Knochenrinde bezeichnet, zur Auflage zu kommen,
wobei die Vorrichtung **dadurch gekennzeichnet Ist, dass** jedes freie Ende der Klauen (9) einen Teil der Auflagefläche (11) bildet und nicht scharfkantig (spitz) ist, sodass das freie Ende dieser Klauen praktisch nicht in die Knochenrinde eindringt.

2. Vorrichtung zur Verankerung eines Muskel/Gewebe-Bandes nach Anspruch 1, **dadurch gekennzeichnet, dass** sie aus zwei Schenkeln (12) besteht, die sich kreuzen unter Bildung eines Ringes bzw. einer Öse.

3. Vorrichtung zur Verankerung eines Muskel/Gewebe-Bandes nach Anspruch 1, **dadurch gekennzeichnet, dass** der Körper der Vorrichtung in der durch die beiden Schenkel definierten Ebene elastisch nicht verformbar ist.

4. Vorrichtung zur Verankerung eines Muskel/Gewebe-Bandes nach Anspruch 1, **dadurch gekennzeichnet, dass** die Schenkel (12) des Körpers eine elastische Einrichtung (13) aufweisen, um eine momentane Annäherung der die Klauen tragenden Enden und das Anordnen des Körpers In einem zylindrischen Raum mit vermindertem Querschnitt zu erlauben, um die Vorrichtung durch die Haut und/oder eine in dem Kopf des Knochens vorgesehene Bohrung hindurch einzuführen.

5. Vorrichtung zur Verankerung eines Muskel/Gewebe-Bandes nach Anspruch 1, **dadurch gekennzeichnet, dass** die Auflageflächen durch Klauen (9) mit kreisförmigem Querschnitt dargestellt sind.

## Claims

1. Device (3) for anchoring a ligament (1) on a bone structure (2), comprising a long-lined body (4), one end (5) of which has a means (6) of hooking on to the bone structure and the other end (7) of which has a ring (8) for fixing the ligament to it at least indirectly, this device being **CHARACTERISED in that**:
- the hooking-on means (6) comprising at least two lugs (9) extending along diverging directions and secant to the longitudinal axis (10) of the body (4) of the device, and each lug (9) is borne by the end of a long-lined arm (12) and the long-lined arms constituting the body of the device gradually separate from each other from the ring (8) with which they are associated, and
- each of the lugs (9) has a supporting face (11) turned towards the ring (8), going on to being supported on the outer face of the bone structure, called cortical, this device being **characterised in that** each free end of the lugs (9) forms a part of the supporting face (11) and is not sharp and so the free end of these lugs can hardly penetrate into the cortical at all.

2. Device for anchoring a ligament according to Claim 1, **characterised in that** it consists of two arms (12) which cross each other to form the ring.

3. Device for anchoring a ligament according to Claim 1, **characterised in that** the body of the device cannot be elastically deformed in the plane defined by the two arms.

4. Device for anchoring a ligament according to Claim 1, **characterised in that** the arms (12) of the body comprise an elastic means (13) to enable the ends with the lugs to come closer together momentarily and house everything in a cylindrical space of reduced section in order to insert the device through the skin and/or through a drilling made in the head of the bone.

5. Device for anchoring a ligament according to Claim 1, **characterised in that** the supporting faces are presented by lugs (9) which are circular in section.
